# EUROPEAN PATENT SPECIFICATION

(11) **EP 4 429 610 B1**
(45) Date of publication and mention of the grant of the patent: **17.06.2026**
(21) Application number: 22817810.9
(22) Date of filing: 08.11.2022
(51) Int. Cl.: A61F 9/007, A61F 13/38, A61F 13/40, A61F 9/00

(54) **KIT FOR REMOVING A DEPOSIT FROM AN EYE**
KIT ZUM ENTFERNEN EINER ABLAGERUNG VON EINEM AUGE
KIT POUR RETIRER UN DÉPÔT D'UN OEIL

(30) Priority: 08.11.2021 IT 202100028352
(43) Date of publication of application: 18.09.2024
(73) Proprietor: Iromed Group S.r.l., 00144 Roma (RM) (IT); Groupadvance Consulting GmbH, 6300 Zug (CH)
(72) Inventor: PONTECORVO, Carmine, 80073 Capri NA (IT)
(74) Representative: Barbaro, Gaetano
(86) International application number: PCT/IB2022/060714
(87) International publication number: WO 2023/079532

(56) References cited:
- EP-B1- 2 877 134
- US-A- 4 875 602

## Description

### Technical field of the invention

The present description relates to an instrument for removing a deposit from an eye during the treatment of an eye disorder to be used independently or in conjunction with a motorized cleaning device, as well as a disposable kit comprising such an instrument and a single-dose container filled with medical liquid.

### Background

It is known from EP2877134B1 a cleaning instrument for removing a deposit from an eye during the treatment of an ocular disorder such as, for example, blepharitis, meibomitis and dry eye syndrome.

The cleaning instrument comprises a swab made of a spongy material adapted to contact a human eye. Such a swab has a tip portion, sized to remove deposits on an eyelid margin of an eye, and a base portion. Furthermore, the cleaning instrument comprises a rigid element which has a distal end portion fixed to the base portion of the swab and a proximal end portion shaped so that it can be coupled to a mandrel of a power-driven cleaning device. In this way, the cleaning instrument can be driven into rotation when the mandrel is rotated.

Before use, the swab of the cleaning instrument must be soaked in a substance suitable for cleaning the eye rim. In order to do this, it is common practice to pour a certain amount of this liquid from a container into a tray. Subsequently, the swab is immersed inside such tray in order to adequately imbibe the swab.

However, this operation exposes the liquid, and consequently the swab, to the risk of contamination. In fact, while using the cleaning instrument, it may be necessary to further soak the swab. The user then dips the swab back into the tray, contaminating the liquid therein with pathogens from the eye being treated. Therefore, a sterilization or a replacement of the bowl is necessary before a new treatment with the cleaning instrument.

Alternatively, it is also possible to directly pour cleaning liquid directly from the original container onto the swab. However, this method is not commonly used due to the difficulty, for example, of uniformly wetting the swab or not dispersing large quantities of liquid.

The US patent No. 4875692, upon the disclosure of which the preamble portion of the claim is drafted, disclosed a liquid dispensing apparatus comprising an absorbent tip, like a sponge, directly fixed upon a distal portion of a pipette.

### Summary of the invention

The purpose of this description is to provide a cleaning instrument which solves the issues of the state of the art. This purpose is achieved with the kit according to claim 1 and the dependent claims.

### Brief description of the figures

Further advantages and characteristics of the cleaning instrument according to the present description will become apparent to those skilled in the art from the following detailed and non-limiting description of an embodiment thereof with reference to the accompanying drawings in which:
- figure 1 shows an isometric view of a cleaning instrument according to a first embodiment;
- figure 2 shows an exploded view of the cleaning instrument of figure 1;
- figure 3 shows a sectional view of the cleaning instrument of figure 1;
- figures 4a and 4b show two moments of the soaking process of a swab of the cleaning instrument of figure 1;
- figure 5 shows a motorized cleaning device to which a cleaning instrument of figure 1 is associated.
- figure 6 shows a detail of figure 5; And
- figure 7 shows an isometric view of a cleaning instrument according to a second embodiment.

### Detailed description of preferred embodiments

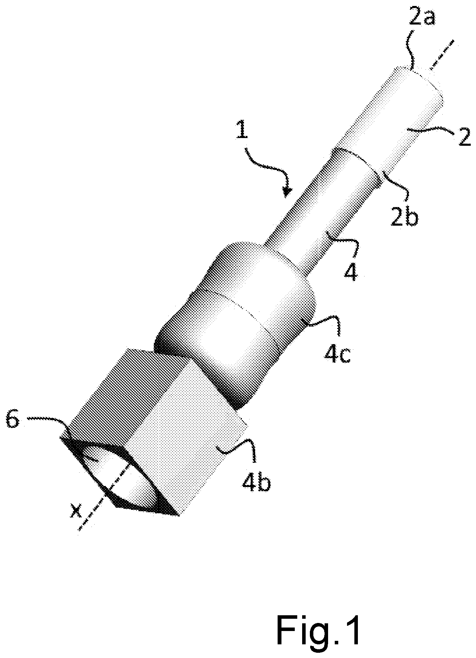

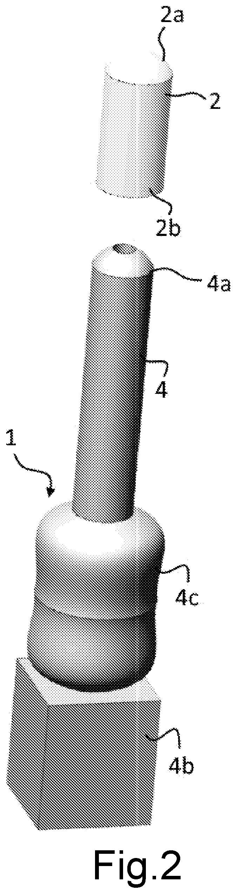

Referring to figures 1-7, an instrument 1 for removing a deposit from an eye during the treatment of an eye disorder comprises a swab 2 and a rigid element 4.

The swab 2 is made of a spongy material, preferably sterile, suitable for coming into contact with a human eye, for example a sponge made of PVA material. Swab 2 has a tip portion 2a and a base portion 2b. The tip portion 2a is sized to remove deposits on an eyelid margin of an eye.

The rigid element 4, preferably made of plastic material, has a main development along a longitudinal axis x and has a distal end portion 4a and a proximal end portion 4b along said longitudinal axis x. The base portion 2b of the swab is fixed to the distal end portion 4a of said rigid element 4 so as to at least partially cover the distal end portion 4a of the rigid element 4 to prevent it from accidentally coming into contact with the eyelid. Preferably, the rigid element is a cylindrical body which develops along the longitudinal axis x and the base portion 2b of the swab 2 comprises a mouth with a circular section suitable for receiving the distal end portion 4a of the rigid element 4.

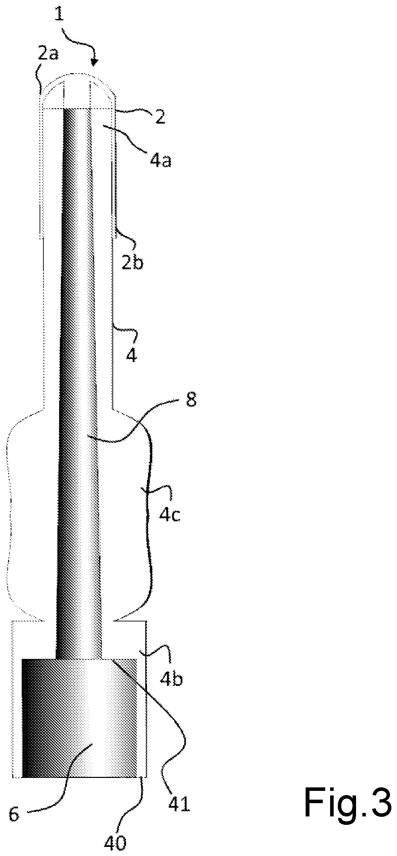

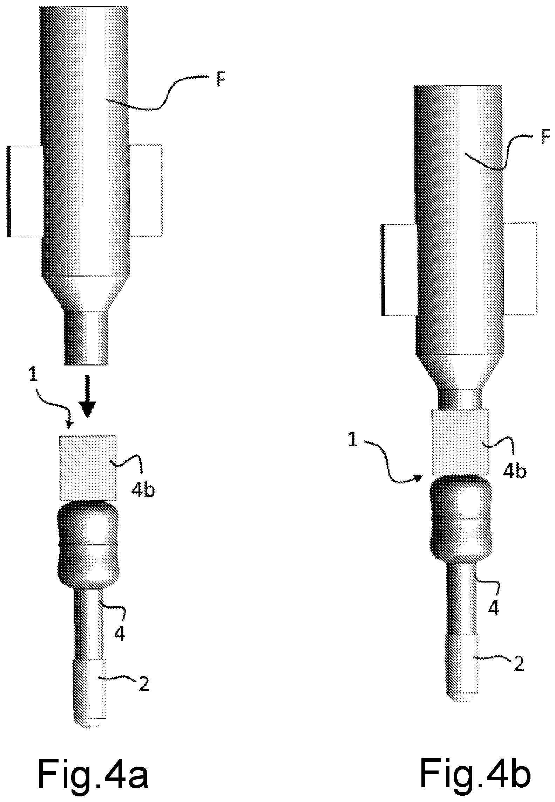

Advantageously, the rigid element 4 comprises, at the proximal end portion 4b, a seat 6 configured to receive in entrance a neck of a container F for containing and transporting medical liquids. An example of such a container F, a disposable plastic vial, is shown for example in figures 4a 4b. With reference in particular to figure 3, the rigid element 4 defines inside it a channel 8, preferably directed according to the longitudinal axis x and configured to put the seat 6 in fluid communication with the swab 2 so that a liquid injected into said channel from said container F, by gravity and/or compression exerted on said container by a user, can flow in the channel of said rigid element 4 from the proximal end portion 4b to swab 2 to soak said swab 2.

The seat 6 has a minimum cross section, with respect to the longitudinal axis x, having an area greater than the area of a maximum cross section, with respect to said longitudinal axis x, of the channel 8.

Thanks to this characteristic, it is possible to insert the neck of the container F inside the seat 6 even when said neck is wider than the maximum cross section of the channel 8. In fact, the channel 8 has maximum dimensions limited by the fact that the distal end 4a of the rigid element 4 which contains the channel 8 must be covered by a swab 2 of suitable size for use in cleaning the edge of the eye, while the neck of the container F has minimum dimensions limited by the construction standards of these containers. Therefore, if the seat 6 were not significantly larger in size than the channel 8, it would not be possible to insert the neck of the container F inside the seat 6, forcing the user to try to pour liquid into an opening of inevitably very limited dimensions.

Preferably, the proximal end portion 4b of the rigid element 4 has a bottom wall 41 which forms the bottom of the seat 6 and which extends perpendicular to the channel 8. The channel 8 in turn develops starting from a central portion of the bottom wall 41. Thanks to this feature, not only can the neck of the container F penetrate inside the seat 6, but it is also possible for the neck to abut against the bottom wall 41 to improve the coupling between the container F and the seat 6 Furthermore, when the neck of the container F rests on the bottom wall 41, an internal cavity of the container F containing the liquid can enter into fluid communication with the channel 8 of the instrument 1 minimizing the loss of liquid during the transfer and making it faster transfer.

Preferably, the rigid element 4 is a hollow cylindrical body and the channel 8 is a through channel with a circular section, through from the proximal end portion 4b to the distal end portion 4a. The seat 6 is a cavity having a circular section, for example of a frustoconical shape, preferably a substantially cylindrical cavity, and its circular section, at any point of the longitudinal axis x, has a diameter greater than the diameter of the channel (8), measured at any point on the longitudinal axis x.

With reference to figures 4a and 4b, the steps necessary to soak the swab 2 of the cleaning instrument 1 can be observed: first of all, see figure 4a, it is necessary to insert the neck of a container of liquid inside the seat 6 defined in correspondence with the proximal end portion 4b of the rigid element 4. Subsequently, see figure 4b, it is necessary to make the liquid flow, by gravity, and/or by compressing the walls of the container, inside the seat 6. The liquid will flow, again due to the effect of gravity and/or of the pressure applied, from the seat 6, along the channel 8, up to the swab 2.

Thanks to the characteristics of the instrument 1 it is therefore possible to soak the swab 2 without using a tray as in the state of the art. In other words, it is not necessary to fill a special container to soak the swab 2 before using the instrument 1 thus reducing the risk of contamination.

Advantageously, the diameter of the channel 8 is such that, when the swab 2 is soaked in liquid, there is a capillary effect which prevents the return of liquid from the swab 2 towards the seat 6 due to gravity.

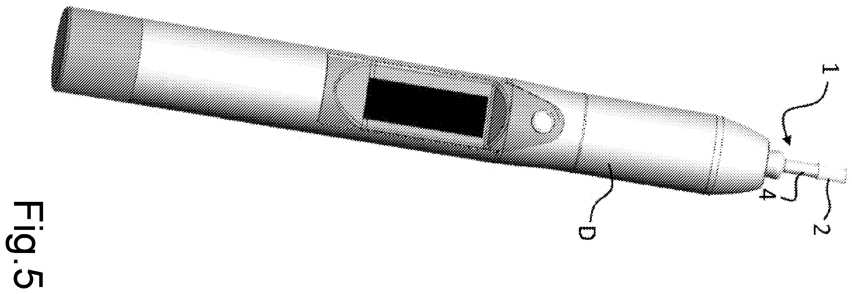

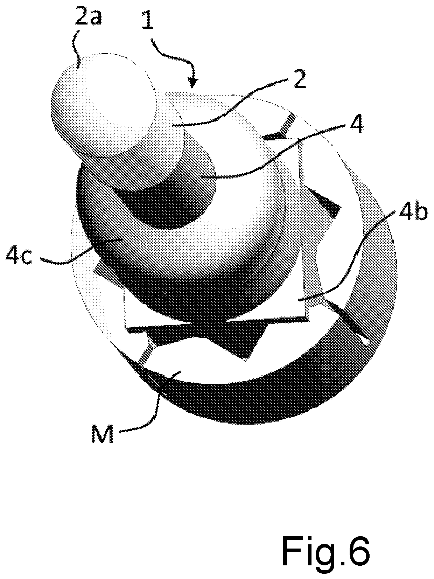

With reference to figures 1, 2, 3, 4a, 4b, 5 and 6, the proximal end portion 4b of a cleaning instrument according to a first embodiment is shaped in such a way that it can be coupled to a mandrel of a motorized cleaning device so as to be driven into rotation when said mandrel is rotated. Preferably, the proximal end portion 4b has an external conformation substantially like a right prism, i.e., a polyhedron whose bases are two congruent polygons placed on parallel planes and connected by a cycle of rectangles. This proximal end portion 4b shaped like a right prism has a proximal base 40 which extends perpendicular to the longitudinal axis x. The seat 6 opens starting from the proximal base 40 of the proximal end portion 4b shaped like a right prism. More particularly, the proximal end portion 4b is preferably parallelepiped-shaped. This right prism shape, or even better parallelepiped shape, contrary to the shape of the state-of-the-art cleaning instrument, is particularly advantageous as it allows the proximal end portion 4b of the rigid element to perform the dual function of coupling with a mandrel and body within which the seat 6 is obtained. Figure 5 shows a motorized cleaning device D, known per se, to which a cleaning instrument 1 is associated. Figure 6 shows a mandrel M of the cleaning device D inside which the proximal end portion 4b of the cleaning instrument 1 is inserted. The rigid element 4 of a cleaning instrument 1 according to the first embodiment preferably comprises a central portion 4c which has a bulge, preferably with an ergonomic shape to be gripped with two fingers, so that the cleaning instrument is easier to grip during the soaking operation of the swab 2 or the insertion of the proximal end portion inside the mandrel M.

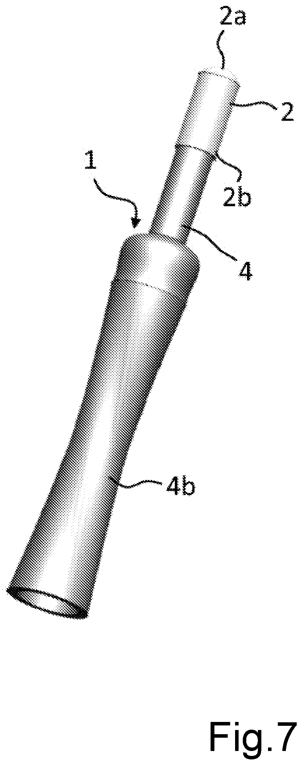

With reference to figure 7, the proximal end portion 4b of a cleaning instrument according to a second embodiment is shaped and sized in such a way so that it is possible to grip the instrument 1 with one hand to manually remove deposits on an eyelid margin of an eye without the use of a motorized cleaning device. For this purpose, the proximal end portion 4b is substantially elongated with respect to the longitudinal axis x so as to allow easier manoeuvring of the cleaning instrument 1.

An example of an eye deposit removal kit includes:
- an instrument 1 for removing a deposit from an eye during the treatment of an eye disorder, comprising:
- a swab 2 made of a spongy material suitable for coming into contact with a human eye, said swab 2 having a tip portion 2a and a base portion 2b, said tip portion 2a being sized to remove deposits on an eyelid margin of an eye; and
- a rigid element 4 having a main development along a longitudinal axis x, said rigid element 4 having along said longitudinal axis x a distal end portion 4a and a proximal end portion 4b;
   wherein said base portion 2b of said swab 2 is fixed to said rigid element 4 so as to at least partially cover said distal end portion 4a of the rigid element 4 to prevent it from accidentally coming into contact with the eyelid;
   wherein said rigid element 4 comprises, at said proximal end portion 4b, a seat 6 configured to receive in entrance a neck of a container F for containing and transporting medical liquids,
   and wherein said rigid element 4 defines inside it a channel 8 configured to put said seat 6 into fluid communication with said swab 2 so that a liquid injected into said channel 8 from said container F, by gravity and/or pressure exerted on said container F by a user, can flow in said channel 8 of said rigid element 4 from the proximal end portion 4b to the swab 2 to soak said swab 2;
- one or more single-dose containers F filled with medical liquid which have a neck configured to penetrate inside the seat 6 of the instrument 1;
- a package containing said instrument 1 and said single-dose container F.

Possible variations or additions may be made by those skilled in the art to the embodiments described and illustrated herein, remaining within the scope of the following claims.

## Claims

1. A kit for the removal of a deposit from one eye, comprising:
- an instrument (1) for removing a deposit from an eye during the treatment of an eye disorder, comprising:
- a swab (2) made of a spongy material suitable for coming into contact with a human eye, said swab (2) having a tip portion (2a) and a base portion (2b), said tip portion (2a) being sized to remove deposits on an eyelid margin of an eye; and
- a rigid element (4) having a main development along a longitudinal axis (x), said rigid element (4) presenting along said longitudinal axis (x) a distal end portion (4a) and a proximal end portion (4b);
wherein said base portion (2b) of said swab (2) is fixed to said rigid element (4) so as to at least partially cover said distal end portion (4a) of the rigid element (4) to prevent it from being accidentally in contact with the eyelid;
wherein said rigid element (4) comprises at said proximal end portion (4b) a seat (6) configured to receive in entrance a neck of a container (F) designed to contain and transport medical liquids;
and wherein said rigid element (4) defines inside it a channel (8) configured to put said seat (6) in fluid communication with said swab (2) so that a liquid injected into said channel (8) from said container (F), by gravity and/or compression exerted on said container (F) by a user, can flow in said channel (8) of said rigid element (4) from the proximal end portion (4b) to the swab (2) to soak said swab (2);
wherein said seat (6) has a minimum cross section, with respect to said longitudinal axis (x), having an area greater than the area of a maximum cross section, with respect to said longitudinal axis (x), of said channel (8), so that said seat (6) can receive said neck of said container (F) even when said neck is wider than the maximum cross section of the channel (8);
**characterized in that** said proximal end portion (4b) has an external shape substantially in the form of a right prism, having a proximal base (40) which extends perpendicular to said longitudinal axis (x), so that said proximal end portion (4b) can be coupled to a mandrel (M) of a motorized cleaning device (D) so as to be driven into rotation when said mandrel (M) is rotated, said seat (6) opening from said proximal base (40) of said proximal end portion (4b) shaped like a right prism;
said kit further comprising:
- a motorized cleaning device (D) comprising a mandrel (M) configured to be coupled with said proximal end portion (4b) shaped like a right prism of said instrument (1), wherein said mandrel (M) is shaped so that said proximal end portion (4b) can be inserted inside the mandrel (M).

2. The kit according to claim 1, wherein said proximal end portion (4b) has a bottom wall (41) which forms the bottom of said seat (6) and which extends substantially perpendicular to said longitudinal axis (x), said channel (8) in turn developing from a central portion of said bottom wall (41).

3. The kit according to claim 1 or 2, wherein said rigid element (4) is a hollow cylindrical body, and the channel (8) has a circular cross-section and is a through channel from the proximal end portion (4b) to the distal end portion (4a), wherein also said seat (6) has a circular cross-section, and wherein said seat (6) has a circular cross-section with a diameter greater than the diameter of the circular cross-section of said channel (8).

4. The kit according to claim 1, wherein said channel (8) is a channel with a diameter such that the capillary effect prevents the return of liquid from the swab (2) to the seat (6) due to the effect of gravity.

5. The kit according to any one of the preceding claims wherein said base portion (2b) of the swab (2) comprises a mouth with a circular section adapted to receive the distal end portion (4a) of the rigid element (4).

6. The kit according to claim 1 or claim 4, wherein said proximal end portion (4b) is substantially parallelepiped in shape.

7. The kit according to any one of the preceding claims, wherein said rigid element (4) comprises a central portion (4c) which has a bulge with an ergonomic shape to be gripped with two fingers.

8. The kit according to any one of the preceding claims, wherein said swab (2) is made of PVA.

9. The kit according to claim 1, wherein said proximal end portion (4b) is shaped and sized in such a way that a user can grip the instrument (1) with one hand to manually remove deposits on an eyelid margin of an eye.

10. The kit according to one of the preceding claims, comprising:
- one or more single-dose containers (F) filled with medical liquid which have a neck configured to penetrate inside the seat (6) of the instrument (1);
- a package containing said instrument (1) and said single-dose container (F).

11. The kit according to claim 1, wherein said instrument (1) is according to claim 2 or any one of claims 3-8 when dependent on claim 2, wherein said neck of said single-dose container (F) is configured to rest on said bottom wall (41) and in such a way that an internal cavity of said single-dose container (F), when said neck rests on said bottom wall (41), is in fluid communication with said channel (8) of said instrument (1).

## Patentansprüche

1. Kit für die Entfernung einer Ablagerung aus einem Auge, umfassend:
- ein Instrument (1) zum Entfernen einer Ablagerung aus einem Auge während der Behandlung einer Augenerkrankung, umfassend:
- einen Tupfer (2), der aus einem schwammartigen Material hergestellt ist, das geeignet ist, mit einem menschlichen Auge in Kontakt zu kommen, wobei der Tupfer (2) einen Spitzenabschnitt (2a) und einen Basisabschnitt (2b) aufweist, wobei der Spitzenabschnitt (2a) dimensioniert ist, um Ablagerungen an einem Lidrand eines Auges zu entfernen; und
- ein starres Element (4), das einer Haupterstreckung entlang einer Längsachse (x) aufweist, wobei das starre Element (4) entlang der Längsachse (x) einen distalen Endabschnitt (4a) und einen proximalen Endabschnitt (4b) vorweist;
wobei der Basisabschnitt (2b) des Tupfers (2) an dem starren Element (4) befestigt ist, um den distalen Endabschnitt (4a) des starren Elements (4) mindestens teilweise zu bedecken, um zu verhindern, dass er versehentlich mit dem Augenlid in Kontakt kommt;
wobei das starre Element (4) an dem proximalen Endabschnitt (4b) einen Sitz (6) umfasst, der konfiguriert ist, um bei einer Einführung einen Hals eines Behälters (F) aufzunehmen, der ausgelegt ist, um medizinische Flüssigkeiten zu enthalten und zu transportieren;
und wobei das starre Element (4) in seinem Inneren einen Kanal (8) definiert, der konfiguriert ist, um den Sitz (6) in Fluidverbindung mit dem Tupfer (2) zu bringen, so dass eine Flüssigkeit, die in den Kanal (8) aus dem Behälter (F) injiziert wird, durch Schwerkraft und/oder Kompression, die durch einen Benutzer auf den Behälter (F) ausgeübt wird, in dem Kanal (8) des starren Elements (4) von dem proximalen Endabschnitt (4b) zu dem Tupfer (2) fließen kann, um den Tupfer (2) zu tränken;
wobei der Sitz (6) einen minimalen Querschnitt hinsichtlich der Längsachse (x) aufweist, der eine Fläche aufweist, die größer als die Fläche eines maximalen Querschnitts hinsichtlich der Längsachse (x) des Kanals (8) ist, so dass der Sitz (6) den Hals des Behälters (F) aufnehmen kann, selbst wenn der Hals breiter als der maximale Querschnitt des Kanals (8) ist;
**dadurch gekennzeichnet, dass** der proximale Endabschnitt (4b) eine äußere Form im Wesentlichen in der Form eines geraden Prismas aufweist, der eine proximale Basis (40) aufweist, die sich senkrecht zu der Längsachse (x) erstreckt, so dass der proximale Endabschnitt (4b) mit einem Dorn (M) einer motorisierten Reinigungsvorrichtung (D) gekoppelt werden kann, um in Rotation angetrieben zu werden, wenn der Dorn (M) rotiert wird, wobei sich der Sitz (6) von der proximalen Basis (40) des proximalen Endabschnitts (4b) öffnet, der wie ein gerades Prisma geformt ist;
das Kit ferner umfassend:
- eine motorisierte Reinigungsvorrichtung (D), umfassend einen Dorn (M), der konfiguriert ist, um mit dem proximalen Endabschnitt (4b), der wie ein rechtes Prisma geformt ist, des Instruments (1) gekoppelt zu werden, wobei der Dorn (M) geformt ist, so dass der proximale Endabschnitt (4b) in den Dorn (M) eingeführt werden kann.

2. Kit nach Anspruch 1, wobei der proximale Endabschnitt (4b) eine Bodenwand (41) aufweist, die den Boden des Sitzes (6) ausbildet und die sich im Wesentlichen senkrecht zu der Längsachse (x) erstreckt, wobei sich der Kanal (8) wiederum von einem zentralen Abschnitt der Bodenwand (41) entwickelt.

3. Kit nach Anspruch 1 oder 2, wobei das starre Element (4) ein hohler zylindrischer Körper ist, und der Kanal (8) einen kreisförmigen Querschnitt aufweist und ein Durchgangskanal von dem proximalen Endabschnitt (4b) zu dem distalen Endabschnitt (4a) ist, wobei auch der Sitz (6) einen kreisförmigen Querschnitt aufweist, und wobei der Sitz (6) einen kreisförmigen Querschnitt mit einem Durchmesser aufweist, der größer als der Durchmesser des kreisförmigen Querschnitts des Kanals (8) ist.

4. Kit nach Anspruch 1, wobei der Kanal (8) ein Kanal mit einem Durchmesser ist, derart, dass die Kapillarwirkung die Rückkehr von Flüssigkeit von dem Tupfer (2) zu dem Sitz (6) aufgrund der Schwerkraftwirkung verhindert.

5. Kit nach einem der vorstehenden Ansprüche, wobei der Basisabschnitt (2b) des Tupfers (2) eine Öffnung mit einem kreisförmigen Querschnitt umfasst, die angepasst ist, um den distalen Endabschnitt (4a) des starren Elements (4) aufzunehmen.

6. Kit nach Anspruch 1 oder 4, wobei der proximale Endabschnitt (4b) im Wesentlichen parallelepipedförmig ist.

7. Kit nach einem der vorstehenden Ansprüche, wobei das starre Element (4) einen zentralen Abschnitt (4c) umfasst, der eine Wölbung mit einer ergonomischen Form aufweist, um mit zwei Fingern gegriffen zu werden.

8. Kit nach einem der vorstehenden Ansprüche, wobei der Tupfer (2) aus PVA hergestellt ist.

9. Kit nach Anspruch 1, wobei der proximale Endabschnitt (4b) auf eine Weise geformt und dimensioniert ist, dass ein Benutzer das Instrument (1) mit einer Hand greifen kann, um Ablagerungen auf einem Lidrand eines Auges manuell zu entfernen.

10. Kit nach einem der vorstehenden Ansprüche, umfassend:
- einen oder mehrere Einzeldosisbehälter (F), die mit medizinischer Flüssigkeit gefüllt sind, die einen Hals aufweisen, der konfiguriert ist, um in den Sitz (6) des Instruments (1) einzudringen;
- eine Verpackung, die das Instrument (1) und den Einzeldosisbehälter (F) enthält.

11. Kit nach Anspruch 1, wobei das Instrument (1) nach Anspruch 2 oder einem der Ansprüche 3 bis 8, in Abhängigkeit von Anspruch 2, ist, wobei der Hals des Einzeldosisbehälters (F) konfiguriert ist, um auf der Bodenwand (41) zu ruhen und auf eine Weise, dass ein innerer Hohlraum des Einzeldosisbehälters (F), wenn der Hals auf der Bodenwand (41) ruht, in Fluidverbindung mit dem Kanal (8) des Instruments (1) ist.

## Revendications

1. Kit destiné à l'élimination d'un dépôt d'un œil, comprenant :
- un instrument (1) permettant d'éliminer un dépôt d'un œil au cours du traitement d'un trouble oculaire, comprenant :
- un écouvillon (2) constitué d'un matériau spongieux approprié pour entrer en contact avec un œil humain, ledit écouvillon (2) ayant une partie de pointe (2a) et une partie de base (2b), ladite partie de pointe (2a) étant dimensionnée pour éliminer des dépôts sur le bord d'une paupière d'un œil ; et
- un élément rigide (4) ayant un développement principal le long d'un axe longitudinal (X), ledit élément rigide (4) présentant le long dudit axe longitudinal (X) une partie d'extrémité distale (4a) et une partie d'extrémité proximale (4b) ;
dans lequel ladite partie de base (2b) dudit écouvillon (2) est fixée audit élément rigide (4) de manière à couvrir au moins partiellement ladite partie d'extrémité distale (4a) de l'élément rigide (4) pour l'empêcher d'être accidentellement en contact avec la paupière ;
dans lequel ledit élément rigide (4) comprend, au niveau de ladite partie d'extrémité proximale (4b), un siège (6) conçu pour recevoir en entrée un col d'un récipient (F) conçu pour contenir et transporter des liquides médicaux ;
et dans lequel ledit élément rigide (4) définit à l'intérieur de celui-ci un canal (8) conçu pour mettre ledit siège (6) en communication fluidique avec ledit écouvillon (2) de sorte qu'un liquide injecté dans ledit canal (8) depuis ledit récipient (F), par gravité et/ou compression exercée sur ledit récipient (F) par un utilisateur, puisse s'écouler dans ledit canal (8) dudit élément rigide (4) depuis la partie d'extrémité proximale (4b) jusqu'à l'écouvillon (2) pour tremper ledit écouvillon (2) ;
dans lequel ledit siège (6) a une section transversale minimale, par rapport audit axe longitudinal (x), ayant une surface supérieure à la surface d'une section transversale maximale, par rapport audit axe longitudinal (x), dudit canal (8), de sorte que ledit siège (6) peut recevoir ledit goulot dudit récipient (F) même lorsque ledit goulot est plus large que la section transversale maximale du canal (8) ;
**caractérisé en ce que** ladite partie d'extrémité proximale (4b) a une forme externe sensiblement en forme de prisme droit, ayant une base proximale (40) qui s'étend perpendiculairement audit axe longitudinal (X), de sorte que ladite partie d'extrémité proximale (4b) peut être accouplée à un mandrin (M) d'un dispositif de nettoyage motorisé (D) de manière à être entraîné en rotation lorsque ledit mandrin (M) est mis en rotation, ledit siège (6) s'ouvrant à partir de ladite base proximale (40) de ladite partie d'extrémité proximale (4b) ayant la forme d'un prisme droit ;
ledit kit comprend en outre :
- un dispositif de nettoyage motorisé (D) comprenant un mandrin (M) conçu pour être accouplé à ladite partie d'extrémité proximale (4b) en forme de prisme droit dudit instrument (1), dans lequel ledit mandrin (M) est formé de sorte que ladite partie d'extrémité proximale (4b) peut être insérée à l'intérieur du mandrin (M).

2. Kit selon la revendication 1, dans lequel ladite partie d'extrémité proximale (4b) a une paroi inférieure (41) qui forme le fond dudit siège (6) et qui s'étend sensiblement perpendiculairement audit axe longitudinal (x), ledit canal (8) se développant à son tour à partir d'une partie centrale de ladite paroi inférieure (41).

3. Kit selon la revendication 1 ou 2, dans lequel ledit élément rigide (4) est un corps cylindrique creux, et le canal (8) a une section transversale circulaire et est un canal traversant de la partie d'extrémité proximale (4b) à la partie d'extrémité distale (4a), dans lequel également ledit siège (6) a une section transversale circulaire, et dans lequel ledit siège (6) a une section transversale circulaire avec un diamètre supérieur au diamètre de la section transversale circulaire dudit canal (8).

4. Kit selon la revendication 1, dans lequel ledit canal (8) est un canal avec un diamètre tel que l'effet capillaire empêche le retour de liquide de l'écouvillon (2) vers le siège (6) en raison de l'effet de la gravité.

5. Kit selon l'une quelconque des revendications précédentes, dans lequel ladite partie de base (2b) de l'écouvillon (2) comprend une embouchure avec une section circulaire adaptée pour recevoir la partie d'extrémité distale (4a) de l'élément rigide (4).

6. Kit selon la revendication 1 ou la revendication 4, dans lequel ladite partie d'extrémité proximale (4b) est de forme sensiblement parallélépipédique.

7. Kit selon l'une quelconque des revendications précédentes, dans lequel ledit élément rigide (4) comprend une partie centrale (4c) qui a un renflement de forme ergonomique pour être saisi avec deux doigts.

8. Kit selon l'une quelconque des revendications précédentes, dans lequel ledit écouvillon (2) est constitué de PVA.

9. Kit selon la revendication 1, dans lequel ladite partie d'extrémité proximale (4b) est formée et dimensionnée de telle sorte qu'un utilisateur peut saisir l'instrument (1) d'une main pour éliminer manuellement des dépôts sur le bord d'une paupière d'un œil.

10. Kit selon l'une quelconque des revendications précédentes, comprenant :
- un ou plusieurs récipients unidoses (F) remplis de liquide médical dont le col est conçu pour pénétrer à l'intérieur du siège (6) de l'instrument (1) ;
- un emballage contenant ledit instrument (1) et ledit récipient unidose (F).

11. Kit selon la revendication 1, dans lequel ledit instrument (1) est selon la revendication 2 ou l'une quelconque des revendications 3 à 8 lorsqu'elle dépend de la revendication 2, dans lequel ledit goulot dudit récipient à dose unique (F) est conçu pour reposer sur ladite paroi inférieure (41) et de telle manière qu'une cavité interne dudit récipient à dose unique (F), lorsque ledit goulot repose sur ladite paroi inférieure (41), est en communication fluidique avec ledit canal (8) dudit instrument (1).
